# EUROPEAN PATENT SPECIFICATION

(11) **EP 0 526 566 B1**
(45) Date of publication and mention of the grant of the patent: **25.11.1998**
(21) Application number: 91909083.7
(22) Date of filing: 22.04.1991
(51) Int. Cl.: A61F 5/41, A61K 9/02, A61K 9/08, A61K 9/10, A61K 9/22, A61K 47/10

(54) **TREATMENT OF ERECTILE DYSFUNCTION**
BEHANDLUNG VON EREKTIONVERSAGEN
TRAITEMENT DE DYSFONCTIONNEMENT ERECTILE

(30) Priority: 25.04.1990 US 514397
(43) Date of publication of application: 10.02.1993
(73) Proprietor: Vivus, Inc., California 94025 (US)
(72) Inventor: PLACE, Virgil, A., Kawaihae, HI 96743 (US); GALE, Robert, M., Los Altos, CA 94022 (US); BERGGREN, Randall, G., Livermore, CA 94550 (US)
(74) Representative: Evans, David Charles
(86) International application number: US9102758
(87) International publication number: WO9116021

(56) References cited:
- EP-A- 0 346 297
- EP-A- 0 354 992
- EP-A- 0 357 581
- WO-A-90/12586
- US-A- 1 897 423
- US-A- 4 801 587
- US-A- 4 829 991

## Description

This invention relates to the treatment of erectile disfunction and more particularly to the treatment of impotence, priapism and Peyronie's syndrome.

As used herein, the term "erectile dysfunction" refers to certain disorders of the cavernous tissue of the penis and the associated facia which produce impotence, the inability to attain a sexually functional erection; priapism, the persistent and often painful erection of the penis; and Peyronie's syndrome, a condition characterized by fibrosis of the cavernous tissue and associated painful and distorted erection of the penis. Erectile dysfunctions, particularly impotence, affect a substantial number of patients. For example, impotence is estimated to affect approximately 10 million American men and can result from any of numerous physiological or psychological factors which cause the blood flow to and from the penis to remain in balance thereby preventing retention of sufficient blood to cause rigid dilation of the corpus cavernosa and spongiosa. As used herein, the term "impotence" is used in its broadest sense as the inability to attain a sexually functional erection when desired. Treatments for impotence include psychosexual therapy, hormonal therapy, administration of vasodilators such as nitroglycerin and α-adrenergic blocking agents (hereafter "α-blockers"), vascular surgery, implanted penile prostheses, vacuum devices and external aids such as penile splints to support the penis or penile constricting rings to alter the flow of blood through the penis. See Robert J. Krause, et al., Impotence, N. Eng. J. Med. Vol. 321, No. 24, December 14, 1989 for a general discussion of the current state of the art.

In 1980, Dr. R. Verig of Paris first demonstrated that impotence could be physiologically treated by the direct injection of a vasoactive drug into a patient's epigastric artery and thereafter thousands of patients have treated their impotence by self injection of such drugs directly into the corpora cavernosa. Forward, 1^{er} Symposium International Sur L'Erection Pharmacologique, 17-19 November 1989, Paris, p. 2; R. Virag, et al, Intracavernous Injection of Papaverine as a Diagnostic and Therapeutic Method in Erectile Failure, Angiology, 35, pp. 79-87, 1984; (See also, U.S. Patents 4,127,118, 4,766,889 and 4,857,059 which are incorporated herein by reference). The drugs most commonly used include α-blockers, such as the long acting phenoxybenzamine and the short acting phentolamine, smooth muscle relaxants such as papaverine, prostaglandins having a vasoactive function such as prostaglandin-E₁ (PGE₁) and combinations of such drugs having different receptor effects to enhance therapy. Erection producing intracavernous injection doses of papaverine are typically in the range of about 7.5 to 160 mg, of phentolamine are in the range of about 0.1 to 10 mg of and of PGE₁ are in the range of about 2.5 to 50 micrograms. See for example, Kurkle, et al, Injection Therapy for Impotence Urol. Clin. of America, Vol. 15, No. 4, Nov. 88, pp. 625-629 and N. Ishii, et al, Intra Cavernous Injection of Prostaglandin E for the Treatment of Erectile Impotence, J. of Urol., Vol. 141, Feb. 1989, pp. 323-325. Vasoactive intestinal peptides at doses of 10-100 µg have also been reported as producing erection on intracavernous injection. See also, H. Handelsman, Diagnosis and Treatment of Impotence, U.S. Dept. of Health Services, Agency for Health Care Policy and Research, April 1990, for a summary of intracavernal injection and other treatment of impotence.

Although intracavernous injection of vasoactive drugs can produce a relatively rapid onset of erection in patients suffering from impotence attributable to venous leakage or arterial insufficiency; patients often find the injections psychologically disturbing, painful, traumatic or inconvenient as evidenced by a high discontinuance rate. See S. Althouf, et al, Why Do So Many People Drop Out From Auto-Injection Therapy for Impotence?, Journal of Sex & Marital Therapy, Vol. 15, No. 2, 1989, pp. 121-129. Adverse side effects including priapism, corporeal nodules and diffuse fibrosis, drug tolerance, bruising and hematomas. Swelling and ulceration of penile skin at the injection site have also been reported.

Nevertheless, because of the relatively innocuous intervention involved and the high failure rate of penile prostheses, the pharmacological approach to the treatment of impotence is still quite advantageous to a large number of patients and could be even more so if the side effects could be avoided. The administration of vasodilators via the male urethra has been disclosed in Kock, EPA 0357581 to produce erections as has the transurethral administration of testosterone, S. M. Milco, Bulletins et Memoirs de la Societa Roumaine D'Endocrinologie, Vol. 5, pp. 434-437 (Dec. 1939). It has also been suggested that cocaine administered transurethrally could contribute to an erection although the reported side effects were catastrophic, JAMA, Vol. 259, NO. 21, page 3176 (1988).

Priapism is less common than impotence and can be attributed to various causes. It has been associated with diseases producing intravascular agglutination or sludging, such as leukemia, and pharmacological priapism has been reported in a small percentage of patients who are treated for impotence by intracavernous injection. Priapism has been treated by intracavernous injection of vasoconstrictors such as α-adrenergic receptor agonists (hereafter "α-agonist"). The reported effective doses of the α-agonist, phenylephrine, are in the range of about 0.1 to 2 mg.

Peyronie's syndrome is a condition of unknown etiology characterized by fibrosis of the cavernous tissues and painful and distorted erections. The current treatment consists of injection of steroids and other anti-inflammatory agents into the site of the fibrosis.

With respect to administration of drugs directly to the penis, medicated catheters such as described in U.S. Patent 4,640,912 have been used to prevent or treat localized infections and irritation of the urethra and bladder; a nitroglycerin coated, erection inducing condom is disclosed in U.S. Patent 4,829,991; the transurethral administration of certain drugs is suggested in U.S. Patents 4,478,822, 4,610,868, 4,640,912 and 4,746,508; and medicated urethral suppositories, inserts or plugs, typically containing anti-infective agents or spermicides are disclosed in U.S. Patents 1,897,423, 2,584,166, 2,696,209 and 3,373,746, for example. As noted above, Kock and Milco disclose introducing agents into the urethra to induce erections.

US-A-1,897,423 discloses a device for preventing gonorrhea infection in consequence of sexual intercourse; the device includes a soft absorbant portion containing a suitable medicament which can be inserted a few millimetres into the urethra in the glans portion of the penis.

US-A-4801587 discloses an ointment for relieving impotence comprising a primary agent, a carrier and a base. In some embodiments, the ointment can be delivered in the urethra by inserting a catheter into the urethra and thereafter attaching to the catheter a tube which contains the ointment. On squeezing the tube, the ointment is delivered into the urethra through the catheter. Excess ointment is expelled by squeezing the penis or by urination.

According to our invention, we have provided dosage forms for the treatment of erectile dysfunction which are painless, capable of rapidly, safely and effectively producing erection of the penis in the case of impotence, detumescence of the penis in the case of priapism and administration of anti-inflammatory drugs to fibrotic sites in the case of Peyronie's syndrome without the above described adverse side effects and with a high degree of patient acceptability.

We have found that the above-described erectile dysfunctions can be safely and effectively treated by the transurethral administration of the appropriate therapeutic drug or combination of therapeutic drugs (as used herein the term "agent" refers to a drug or a combination of drugs capable of producing the desired therapeutic effect) and have provided penile inserts adapted to be easily and painlessly inserted into the urethra, which inserts carry the appropriate therapeutic agent in an amount sufficient to produce the desired result.

In one preferred embodiment of this invention the therapeutic agent is applied as a coating on a penile insert which may be configured to prevent complete insertion and to facilitate removal.

In another preferred embodiment of this invention, the agent is contained in a gel, cream, ointment or suppository for example which may be deposited in the urethra from a specially designed insert.

This invention and the advantages thereof will be readily apparent from the following description of the invention with reference to the accompanying drawings wherein:
Figure 1 is a cross sectional view of one embodiment of this invention;
Figure 2 is a cross sectional view of another embodiment of this invention;
Figure 3 is an exploded view of a penile insert according to the invention and its container;
Figure 4 is a side view, partly in section, of an inserter/container assembly for introducing a composition containing a dose of agent into the urethra; and
Figure 5 is a top view of the inserter/container of Figure 4.

In its broadest aspect, this invention contemplates the treatment of erectile dysfunction by the transurethral administration of an agent, therapeutically effective with respect to the dysfunction, directly into the blood supplying the corpus cavernosum via the cross circulation with the spongiosa surrounding the urethra. The erectile dysfunctions which may be so treated include impotence, for which the therapeutic agent is one or more drugs capable of producing a vaso-dilatory or other erection inducing effect. Suitable vaso-dilatory agents include nitrates such as nitroglycerin and isosorbide dinitrate, long and short acting α-blockers such as phenoxybenzamine, dibenamine, doxazosin, terazosin, phentolamine, tolazoline, prazosin and trimazosin; adenosine, ergot alkaloids, chlorpromazine, haloperidol, yohimbine, verapamil and other calcium blockers, natural and synthetic vasoactive prostaglandins and analogs thereof such as PGE₁, alprostadil and misoprostol, for example, vasoactive intestinal peptides or any other agent which is capable of producing an erection when administered transurethrally. For example, dopamine agonists such as apomorphine and bromocriptine and opioid antagonists such as naltrexone have been reported to induce erection and they may also be useful according to this invention. See S. Lal et al, Apomorphine: Clinical Studies on Erectile Impotence and Yawning, Prog. Neuro-Psychopharmacology, Vol. 13, 1989, pp. 329-339 and A. Fabbri et al, Endorphines in Male Impotence, Evidence for Naltrexone Stimulation of Erectile Activity in Patient Therapy, Psychoneuroendocrinology, Vol. 14, No. 1 & 2, pp. 89, 103-111.

With respect to priapism, the therapeutic agent may be one or more vasoconstrictor drugs. Suitable vasoconstrictors include α-receptor agonists such as epinephrin, phenylethylamine, norepinephrine, dopamine, metaraminol, phenylephrine, methoxamine, ephedrine, phenylpropanolamine, mephentermine and propylhexedrine, for example, β-blockers such as butoxamine, dichloroisoproterenol, propranolol, alprenolol, bunolol, nadolol, oxprenolol, penbutolol, pindolol, sotalol, timolol, metoprolol, atenolol, acebutolol, bevantolol, pafenolol, and tolamolol, for example, and any other agent which is capable of producing a detumescent effect when applied transurethrally.

With respect to Peyronie's syndrome, the therapeutic agent may be one or more anti-inflammatory drugs such as corticosteroids including cortisone, hydrocortisone, tetrahydrocortisone, prednisone, prednisolone, methylprednisolone, fludrocortisone, desoxycortisol, corticosterone, triamcinolone, paramethasone, betamethasone, dexamethasone and beclomethasone, for example, non-steroidal anti-inflammatories such as salicylic acid, aspirin, diflunisal, methyl salicylate, phenylbutazone, oxyphenbutazone, apazone, phenacetin, acetaminophen, indomethacin, sulindac, mefenamic acid, meclofenamate sodium, tolmetin, ibuprofen, naproxen and fenoprofen, for example, and other drugs such as testosterone which may be capable of producing an anti-inflammatory effect on fibrous tissue within the corpus cavernosum when administered transurethrally.

It is preferred that the agent be rapidly delivered through the urethra in order to bring about a rapid onset of the desired effect. To that end the agent containing material is caused to contact the urethra along a sizable portion, about 2-3 cm, of its length rather than localizing it at one site along the urethra. Also, the agent should be applied past the point where the transition from the epidermal character of the glans has been completed.

In all of the dosage forms contemplated herein, it is desirable that the volume of agent-containing material that is deposited in the urethra remain therein until complete absorption of the agent has occurred and that the material be deposited in a manner that permits relatively rapid absorption of the agent. Volumes in the range of 50-100 mg (approximately 50-100 µl) tended to exhibit observable spillage prior to complete absorption. Accordingly, the amount of drug-containing material retained in the urethra is preferred to be below about 50 µl. Adequate lubrication has been obtained with as little as 5-10 µl of lubricating carriers such as polyethylene glycol (PEG) 1000 and 1450.

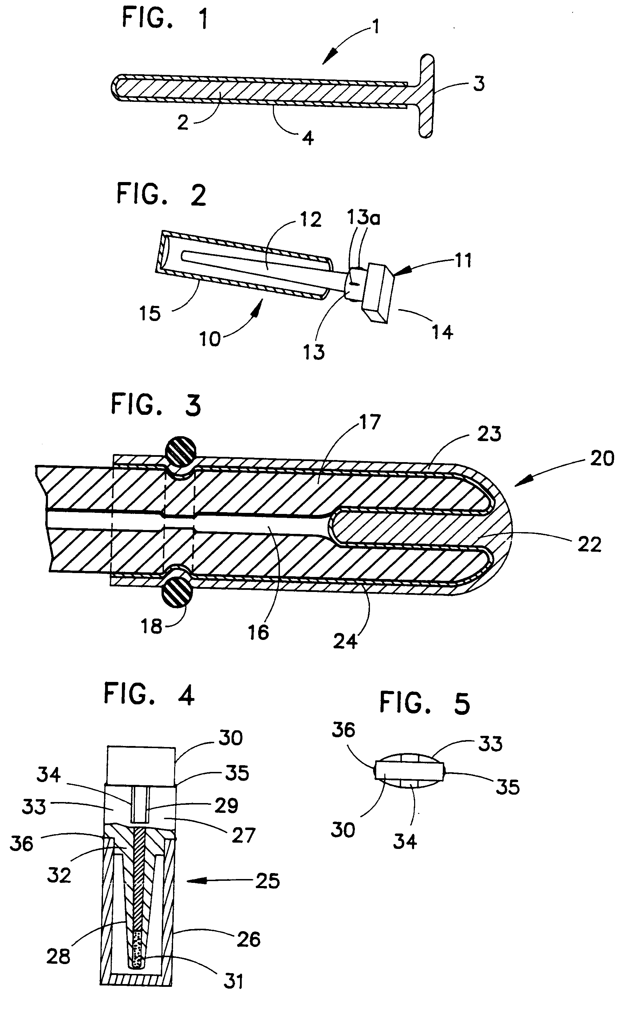

The agent may also be contained in rapidly releasing coatings or suppositories which are rapidly dissolved, melted or bioeroded in the urethra. Urethral permeation enhancers for the agent may also be included in the agent containing composition. In certain embodiments which are illustrated in Figure 1 and 3, the agent is included in a coating on the exterior surface of a penile insert.

In another embodiment illustrated in Figures 4 and 5 the agent is contained in a dose of predetermined volume which is deposited as a suppository into the urethra at the desired location.

Referring now to Figure 1, a penile insert 1 comprises a shaft portion 2 which is sized to be easily and comfortably inserted into the male urethra. Means are also provided to prevent complete insertion of the inserter into the urethra in a manner that would make removal difficult. The means may simply be a portion of the shaft of adequate length to be gripped and not released during use. It is preferable however that the end of shaft 2 is provided with an enlarged terminal portion 3 configured to prevent complete insertion into the urethra and to facilitate removal of the device after the agent has been delivered . The internal end of shaft portion 2 is preferably provided with a rounded, blunted end to prevent discomfort on insertion and is typically from about 3 to 5 millimeters in diameter and from about 2 to 12 centimeters in length.

The insert itself may be made from any pharmacologically acceptable material and although it may be rigid, it is preferred that the device be relatively soft and flexible for purposes of comfort, merely having sufficient rigidity to facilitate insertion. For this purpose various pharmaceutically acceptable natural or synthetic rubber or polymeric materials such as natural rubber, silicone rubber, ethylene vinyl acetate (EVA) copolymers, polyethylene, polypropylene, polycarbonate, polyester, polyurethane, polyisobutylene polymers, and polyoxymethylene polymers such as Delrin® manufactured by Du Pont, for example, are suitable. Polypropylene is particularly useful, especially where the product is to be radiation sterilized.

It is preferable that the agent is concentrated on the urethra-contacting surfaces of the device in order to permit rapid absorption of the agent and any permeation enhancer. As shown in Figure 1, the shaft portion 2 of the insert 1 is provided with an agent-containing coating 4 which comprises the desired agent dose and, if used, a permeation enhancer, dispersed throughout a rapidly releasing carrier. The coating 4 may be applied to the insert by means of dip coating in an appropriate agent-containing bath, spray coating, heat melt coating, evaporation of a fixed volume of a solution or suspension of the agent in a volatile vehicle or by co-extrusion of an agent-containing layer onto the surface of shaft 2, for example.

To facilitate insertion, coating 4 preferably has lubricating properties and may contain dispersant materials such as PEG, propylene glycol, glycerine, polyvinyl pyrrolidine (PVP), polyvinyl alcohol (PVA), hydroxy alkyl celluloses or cyclodextrins, for example, which are or become slippery upon insertion into the urethra. Materials such as glycerol monolaurate, polyethylene glycol monolaurate, and glycerol monolaurate, for example, may combine permeation enhancing properties with lubricating properties.

To facilitate adherence of the drug coatings to the penile insert, the surfaces to which the coatings are applied may be slightly roughened. Also, to provide a visual indication of complete drug release, the coating, instead of being clear and transparent, can be selected to provide a different visual appearance from that of the uncoated insert. This can be accomplished with the use of dyes or pigments or can be a property of the drug or coating material itself.

In use, the device would be inserted slowly (about 5-10 seconds) into the urethra up to the terminal portion 3 and either maintained in place until the agent is absorbed (about 30-45 seconds) and then slowly removed, or more preferably, particularly with shorter devices (about 2-5 cm in length), the device 1 would be inserted into the urethra up to portion 3 and then, while compressing the penis around shaft 2, gently but firmly rotated and reciprocated to wipe all the agent-containing material from the surface of the device prior to removal.

Referring now to Figure 2, a combination insert/container 10 is shown in which the insert 11 is provided with a tapered agent-carrying shaft portion 12 which terminates in a plug portion 13 which may also be provided with sealing ridges 13a. Plug element 13 terminates in cap portion 14 which may be larger than plug 13 and preferably of a square or other polygonal configuration to make it easy to rotate insert 11 for removal from its container 15. Container 15 is generally tubular in shape closed at one end and of sufficient length to receive the insert up to contact with cap 14. The interior diameter of container 15 and the exterior diameter of plug 13 with sealing ridges 13a are selected to provide a sliding seal that is sufficient to prevent insert 1 from falling out of the container and the passage of contaminants into the container while permitting removal of the insert with the application of a reasonable force on cap 14.

Referring now to Figure 3, another embodiment of the invention is shown in which the penile insert 20 comprises a shaft portion 22 adapted to be received within the male urethra and a terminal portion 23 in the form of a tubular cap adapted to enclose the glans and, if more agent delivering surface is required, some portion of the shaft of penis 17. The body-contacting surface of insert 20 is provided with an agent-containing coating 24 similar to that described with respect to Figure 1 which coating is applied to the shaft 22 and such other portion of the interior of the terminal portion 23 as is desired. The embodiment of Figure 3 may be used with respect to less potent agents which require an administration rate greater than can be obtained directly through the urethra. Thus the portion of the coating 24 which contacts the glans and the shaft of the penis also provides for the administration of the agent directly through the skin of the penis in addition to the transurethral administration.

In use, the device would be inserted into the urethra 16 and in contact with the skin of penis 17 and maintained in place until all the agent has been released from coating 14. In Figure 3 a constrictive, typically elastic, band 18 is applied around the base of the penis 17 while the insert 20 is in place to constrict the penis and prevent the flow of blood therefrom. This constrictive band can also be used with respect to the embodiments of Figures 1, 2, 4 and 5. It is useful in impotence where it will prevent the flow of blood from corpus cavernosum thereby assisting in the maintenance of the erection and in Peyronie's syndrome where it will cause the anti-inflammatory drug to remain in the corpus cavernosum for an extended period of time. The constricting bands would not normally be used when the device of this invention is used to treat Priapism where it is desired to increase, rather than retard, the flow of blood from the penis.

Referring now to Figures 4 and 5 another embodiment of this invention is shown for use when the agent is contained in an ointment, paste, suppository, cream or gel formulation of the type described above rather than as a coating on the shaft 28 of an insert. The dosage insert /container 25 comprises a container 26 closed at one end and receiving insert 27 in the other end. Although container 26 can be cylindrical in configuration it is preferred to form container 26 into a more volume efficient flattened configuration such as elliptical or rectangular because there is no need to maintain a large clearance between the exterior of insert 27 and the interior of container 26, to prevent inadvertent removal of any coating on insert 27. Insert 27 comprises a shaft portion 28 having an external configuration similar to that of the insert shown in Figures 1 and 2 but provided with a longitudinal bore which receives the piston portion 29 of plunger 30, the agent-containing dose 31 in the form of an ointment, paste, suppository, cream or gel having sufficient viscosity to enable it to remain without spillage within the cavity formed between the tip of piston 29 and the bore.

Preferably, means are provided to prevent unintentional activation of plunger 30 which in its simplest form could be a frangible bead or bond which resists relative motion of plunger 30 with respect to shaft portion 28 until a predetermined force is applied. A more positive means is illustrated in Figures 4 and 5 wherein shaft portion 28 terminates in a plug portion 32 configured to form a sliding seal with the interior of container 26. The plug portion 32 terminates in cap portion 33 provided with receptacle means 34 configured to receive plunger 30 when plunger 30 is in a first position and to be incapable of receiving plunger 30 when in a second position and being of sufficient depth to allow displacement of piston 29 over sufficient travel to fully displace dose 31 from the insert In Figures 4 and 5 the receptacle 34 is shown as a slot across cap 33. Plunger 30 is mounted transverse to slot 34 and maintained in this first position by a frangible bond 35. Cap 33 is likewise sealed to container 26 by a similar frangible bond 36. These frangible bonds can be formed by any suitable technique which include adhesive bonding, heat or sonic welding or the application of some form of "shrink wrap" material, for example.

This configuration is readily adaptable to automated filling together with precise control of the quantity of agent dose 31 and provides for positive administration of the desired quantity of drug at the desired site of application.

Prior to use the device is protected from inadvertent displacement of dose 31 by means of the frangible seal 35 and inadvertent removal of the insert by means of frangible seal 36. In use, frangible seal 35 would be broken by rotating plunger 30 from its first position to a second position where it is in alignment with receptacle 34 and frangible seal 36 would be broken to remove the insert 27 from container 26. The insert would then be placed into the urethra to the depth of plug 32 and plunger 30 depressed into receptacle 34 to completely eject dose 31 into the urethra at the desired point of application. The insert 27 would then be removed leaving the drug load 31 within the urethra.

The materials used to form the insert /container 25 are the same as those which can be used in fabricating the devices of Figures 1 and 2 for example and when these materials are thermoplastic the formation of the frangible bonds 35 and 36 by sonic fusion is a preferred technique.

The agent in dose 31 can be one or more drugs. However, when a combination of drugs is required to produce the desired therapeutic effect, it is also possible to sequentially administer separate doses of each individual drug, each of which can be titrated by the physician and/or patient to produce the desired effect.

The embodiments of this invention can either be manufactured under sterile conditions thereby eliminating the need for post manufacturing sterilization or they can be manufactured under non-sterile conditions and then subsequently sterilized by any suitable technique such as radiation sterilization.

The penile inserts and injectors of this invention can be manufactured by typical plastic forming and coating process and solvent evaporation known to the art which include molding, extrusion, heat forming, dip coating, spray coating, heat melt coating and solvent evaporation. Although prototype devices of Figure 1 were made from EVA rods which were heat formed by hand into the configuration of Figure 1 on a hot plate to flatten the exterior end and blunt the interior end, components forming the embodiments of Figures 2 and 4 were made in quantity in conventional injection molding equipment.

The molded parts which require coating can be coated by any suitable process. Dip coating, with control of the temperature and viscosity of the bath and the dwell time of the article to be coated in the bath, coupled with air wiping for added precision is capable of producing quite reproducible coatings within the requirements of this invention. Depositing a known volume of a solution or suspension of the agent/dispersant in a volatile carrier onto the shaft in the presence of a warm air stream also produces coating in a reproducible manner as does the application of a known volume of a melt containing the agent to a cool inserter shaft.

PEG based coating formulations are particularly suitable to this invention because they are solid at refrigerator or ambient temperatures but melt at or below body temperature, have lubricating properties and dissolve in the urethra to allow rapid absorption of the active drug therein dispersed. At 70°C the viscosity of a 50-50 blend of PEG 1450 and PEG 400 is such that approximately 100 mg of a dip mixture was reproducibly left on a 10 cm long by 3.5 mm diameter EVA rod after a single dip.

By varying the molecular weight of the PEGs and/or their ratios and/or the temperature of the bath, the viscosity of the bath and the resultant weight of the coating can be adjusted. For example, a 1:2 weight ratio mixture of PEG 600 and PEG 1000 will have a melting point of about 32°C and could be expected to yield a coating of approximately 50 mg at a bath temperature within the range of about 50°-80°C.

PEG 1450 is available as a flake material at room temperature which on melting and cooling forms a smooth coating on the insert of this invention. At bath temperatures in the range of 60°C to 80°C coatings of about 50 mg can be obtained from a single dip.

As another example, at 70°C, PEG 1450 deposited on the 2 cm of the tip equals about 50 mg. The slower dissolution of the higher molecular weight PEG allows controlled deposit on a limited area. As the system is inserted into the urethra the slower melt of PEG 1450 allows minimal deposition in the distal urethra and maximum at the depth of full insertion.

Coatings formed from the lower molecular weight PEG's need to be stored under refrigeration when high ambient temperatures are anticipated. Coatings formed from PEG 1450, however, were physically stable even when carried exposed to high summer temperatures.

Another approach to forming the desired coating on the shaft 12 of the insert is to micropipette a known quantity of the agent and dispersant in solution or suspension in a volatile solvent onto the shaft 12 which is maintained in a downwardly inclined position in the presence of a warm air stream to permit rapid evaporation of the solvent before any of the liquid can drop from the shaft. This approach is particularly suitable when both the agent and the dispersant are mutually soluble in the volatile carrier.

As an example, PGE₁ together with a lubricating dispersant such as propylene glycol, PEG, glycerin, PVP, PVA, hydroxyalkyl cellulose or cyclodextrin, for example, would be dissolved in weight ratios of dispersant to agent of 1:1-10:1 in alcohol at a concentration such that a small volume, approximately 10 µl for example, of the alcoholic solution contains the desired dose of the agent. This predetermined quantity of solution would be introduced from a micropipette onto the surface of the insert in the presence of a warm air stream and would evaporate rapidly to form the desired coating. The volume of solution is not critical but should be selected based on the size of the insert and the viscosity of the solution such that a relatively even coating of the solvent solution on the shaft is obtained without any spillage prior to evaporation of the volatile solvent. Melted PEG containing the agent can also be micropipetted in a similar manner onto a cool insert shaft to provide reproducible doses of single or multiple drugs.

Preferably, the total weight of the coating should be minimized consistent with the maintenance of lubricating properties, and coating weights less than 50 mg are preferred. Similar amounts are also contemplated when the dosage form is in the form of a solution, dispersion, ointment, paste, gel or suppository, for example. At about the 50-100 mg level, the capacity of the urethra to receive the agent containing material and rapidly absorb the agent appears to be reached as some spillage of the coating material was observed.

Once the weight of the agent-containing dose or coating has been selected, the concentration of the agent in the carrier would be appropriately selected to provide the desired total dose within the dosage form or coating.

Unit dosages for PGE₁ are in the range of approximately 10 to 1000 µg, 50 to 500 µg being preferred, the unit dosages of papaverine are in the range of 1-20 mg, and the unit dosages of phentolamine, prazosin and doxazosin are in the range of about 50 to 1000 µg per dose with 100 to 400 µg being preferred. It has been observed that combinations of two or more drugs such as PGE₁ and the α-blockers tend to potentiate the erectile effect thereby permitting efficacy to be obtained at lower doses of both drugs.

If the agent is a combination of drugs they all may be included in one dosage form. However, when the agent comprises more than one drug, it would be preferable to sequentially administer each drug from a dosage form containing only one drug. It is always preferred to use the lowest effective dose in any medical intervention and it is contemplated that the dosage forms of this invention would be provided in various, incremental doses. The patient would initially titrate himself to the dosage effective for him by using the lowest dosage, and repeating administration until the desired effect is obtained. Thereafter the patient would select an effective incremental dosage that is close to the determined higher dosage or could continue using multiple lower doses.

The following examples of this invention are provided.

### EXAMPLE 1

A 3.5 mm EVA (28% VA) rod was formed into an insert having a shaft approximately 10 cm long with a spherical, blunted tip and a head portion approximately 4 mm thick and 1 cm in diameter on a hot plate. A dipping bath comprising a 50-50 weight blend of PEG 1450 and PEG 400 and sufficient agent to attain the desired concentration in 100 mg of coating was prepared and heated to 70°C. The insert suspended by its head, was dipped into the dipping bath and removed.

The total weight of the coating so obtained was approximately 100 mg. Nine inserts having coatings containing approximately 50 µg of PGE₁ and nine inserts having coatings containing approximately 50 µg PGE₁ and 100 µg prazosin hydrochloride were prepared. When used by an impotent human volunteer, four doses of the 50 µg PGE₁ were required to achieve minimal erection. The dosage forms combining PGE₁ and prazosin produced a stronger erection in both normal and impotent volunteers at a lower total dose of two units in a shorter period of time. A slight burning sensation in the urethra was observed by the normal volunteer with the devices using the prazosin hydrochloride but not by the impotent patient. The use of the base form of prazosin rather than the hydrochloride may eliminate the sensation.

### EXAMPLE 2

A 3 mm diameter EVA (24% VA) rod approximately 10 cm in length was blunted and rounded on one end and flattened on the other by manipulation on a hot plate. A gelled aqueous coating formulation, comprising 9 gm of 95% ethanol, 1 gm of propylene glycol, 0.2 gm of hydroxypropyl cellulose and 2 mg of PGE₁ was prepared and dip coated on the shaft of the rod to a total loading of about 1g. The residual ethanol in the coated rod was allowed to evaporate. It is estimated that the loading of PGE₁ was about 200 µg/unit. Penile erection was produced in a normal human subject within ten minutes after the sequential insertion of two rods into the urethra for a total PGE₁ dose of 400 µg.

### EXAMPLE 3

A 3.5 mm EVA (28% VA) rod approximately 10 cm long with rounded tip and flattened head is coated with 500 µg of prazosin base in a PEG blend (1:2 PEG 600:PEG 1000) having a melting point of approximately 32°C. Initial tumescence should be achieved within several minutes after insertion into the male urethra with maximum effect being produced within about fifteen minutes. Both the intensity and duration of effect will be greater in patients with normal vasculature whose impotence is due to neurologic deficiency. With marked vascular damage a single drug may have incomplete action requiring additional doses or the use of a mixed drug formulation.

### EXAMPLE 4

Penile inserts configured as in Example 3 are coated with a mixture of 20 µg of PGE₁ and 200 µg of doxazosin hydrochloride in the PEG mixture described in Example 3. Patients with moderately severe vascular deficiencies should attain erectile response from this dosage in several minutes which should be maintained for approximately thirty minutes.

### EXAMPLE 5

Penile inserts configured as in Example 3 are coated with 100 µg of phenylephrine in approximately 50 mg of the PEG mixture of Example 3 or approximately 100 µg of adrenalin contained in the PEG mixture of Example 3. Inserts so fabricated are inserted into the urethra of a patient suffering from priapism, due either to pharmacological or other causes. If detumescence is not obtained within approximately five to ten minutes the application will be repeated at approximately ten minute intervals until detumescence occurs. Small incremental doses are administered in this fashion to prevent systemic overdosage when balanced circulation is reestablished.

### EXAMPLE 6

Penile inserts configured as in Example 3 are coated with 100 µg of triamcinolone acetonide in approximately 50 mg of the PEG mixture of Example 3 and in 50 mg of PEG in the molecular weight range of about 2000-8000 or 50 µg of fluocinonide in approximately 50 mg of the PEG mixture of Example 3 and in 50 mg of PEG in the molecular weight range of 2000-8000. Such inserts are inserted daily into the urethra of a patient suffering from Peyronie's disease and allowed to remain in the urethra for approximately twenty minutes to provide the steroid in therapeutic local concentration in the corpus cavernosa. This method eliminates the trauma associated with the local injection of the anti-inflammatory agent which may in itself stimulate the fibrotic process which the therapeutic agent is intended to correct. If improvement is not observed after a course of treatment with single doses, multiple doses can be used for twenty minutes each until a dosage level which is effective can be established.

### EXAMPLE 7

Penile inserts as in Figure 1 are dip coated with a mixture of PEG 1450 at a temperature of 70°C containing 1% PGE₁. Dipping to a depth of 2 cm in such a solution leaves a total mass of 50 mg on the distal 2 cm and contains 500 µg of PGE₁. The dipping time is about 1 second and cooling time 3 minutes at room temperature of about 20°C.

This system was stable when carried exposed to summer temperatures in Washington, D.C. and provided adequate lubrication upon insertion when inserted slowly over a period of approximately 5-10 seconds. Approximately 30 seconds after full insertion the system was then slowly removed over 10 seconds, the entire coating had been released into the urethra after this procedure. Erection was obtained after about 15 minutes in a normal male.

### EXAMPLE 8

A penile insert configured as shown in Figure 2 was injection molded to provide a shaft portion 12 approximately 3 cm in length having a taper from approximately 3 mm diameter at the plug end to approximately 2.5 mm at the tip end was injection molded from polyoxymethylene polymer. PGE₁ together with an equal weight amount of propylene glycol as a lubricant/dispersant were dissolved in ethyl alcohol at a concentration of 400 µg PGE₁ per 10 µl of solution. Ten µl of this solution were dispensed from a micropipette onto the shaft portion of the insert, inclined approximately 45° from the vertical, in the presence of warm, flowing air to evaporate the alcohol and provide a thin coating of the PGE₁ in the dispersant on the shaft of the insert. The insert was then placed within its container. The insert was inserted into the urethra and vigorously rotated and reciprocated to wipe the PGE₁ from the surface of the insert for approximately 20 seconds. Similar implants were used by two other subjects and functional erections were obtained in approximately 10 minutes and lasted for approximately 30 minutes. This product exhibited a relatively short shelf life, possibly due to instability of the PGE₁ in the presence of moisture. It would therefore be preferred to manufacture and package the device in a low humidity or nitrogen environment.

### EXAMPLE 9

A penile insert was manufactured as described in Example 8 except that the drug alprostadil (PGE₁) was mixed with a gamma type cyclo- dextrin at a ratio of 1 part alprostadil to 4 parts cyclodextran. This product may have a longer shelf life than the product of Example 8.

### EXAMPLE 10

Inserts configured as shown in Figures 1 and 2 have been evaluated in impotent males in which the impotence is associated with diabetes, post-coronary bypass (vascular insufficiency) and a post-radical prostatectomy, normal aged males and normal healthy males using various vasoactive drugs and combinations thereof. In all instances patients have responded positively although variations in intensity of erection and duration thereof were observed depending on dose, formulation and environment. Analysis of returned used systems showed residual drug loadings of from 0-50% indicating that application technique is important in use of the insert . The preferred insertion technique which is associated with substantially complete drug removal from the inserter involves slow insertion to the maximum depth over a period of 5-10 seconds, followed by reciprocation and rotation of the inserter over approximately a 2-3 cm length while maintaining pressure on the penis to maintain intimate contact between the urethra and the shaft of the insert for about 20 seconds and then slow removal of the insert while maintaining compression on the penis. This technique effectively wipes the surface of the insert clean and provides for substantially complete agent delivery.

### EXAMPLE 11

An insert configured as in Figure 4 was injection molded to provide a shaft portion approximately 3 cm in length having a taper from approximately 3 mm diameter at the plug end to approximately 2.5 mm at the tip end provided with a 1.5 mm central bore. The plunger is inserted into the central bore to a depth that leaves a 1.5 mm long cavity at the tip. The insert is inverted and melted PEG 1450 containing 400 µg of alprostadil is cast into the cavity and solidified therein. Upon solidification, a suppository weighing about 2.9 µg and containing about 15% wt alprostadil was formed. Alternatively, a suppository in the form of a 1.5 mm long cylinder of solid PEG 1450 containing 400 µg of alprostadil could be introduced into the shaft either at the tip or moved to the tip of insert when the plunger is introduced into the 1.5 mm bore. The insert is molded from polypropylene. The insert is then placed within a polypropylene container and a sonic bond is formed between the plug portion of the inserter and the container and between the plunger which is mounted crosswise to the slot in the cap. The device may then be radiation sterilized. In use, the bonds between the plunger and the cap and the cap and the container would be broken and the insert assembly removed from the container, the plunger would be rotated into alignment with the slot in the cap, the shaft of the insert would be inserted into the penis up to the beginning of the plug and the plunger depressed into the slot to inject the agent load into the urethra. The device would then be removed.

Having thus generally described our invention, it is obvious that various modifications will be apparent to workers skilled in the art which can be made without departing from the scope of this invention which is limited only by the following claims wherein:

## Claims

1. A dosage form (1; 10; 20; 25) for treating erectile dysfunction characterised by:-
a) a shaft (2; 12; 22; 28) 2-12cm in length which can be inserted into the urethra of a male patient; and
b) a composition including a preselected therapeutic dose amount of a therapeutic agent for treating erectile dysfunction which composition is disposed in a cavity in the end of the shaft which is inserted into the urethra;
c) said shaft and composition are configured and arranged such that, on insertion of the shaft in to the urethra, the composition is delivered substantially past the point at which the transition from the epidermal character of the glans is complete; and
d) delivery means (29) selectively operable to deliver the composition from the cavity to the urethra.

2. A dosage form (1; 10; 20; 25) for treating erectile dysfunction characterised by:-
a) a shaft (2; 12; 22; 28) 2-12cm in length which can be inserted into the urethra of a male patient; and
b) a coating on at least a portion of the shaft, which coating comprises a composition including a preselected therapeutic dose amount of a therapeutic agent for treating erectile dysfunction; and
c) said shaft and coating are configured and arranged such that, on insertion of the shaft in to the urethra, the composition is delivered substantially past the point at which the transition from the epidermal character of the glans is complete.

3. A dosage form as claimed in claim 1 wherein the said composition further comprises a dispersant for the therapeutic agent.

4. A dosage form as claimed in claim 1, claim 2 or claim 3 and a total amount of the composition does not exceed 100 mg.

5. A dosage form as claimed in any preceding claim wherein the total amount of the composition does not exceed 50 mg.

6. A dosage form as claimed in any preceding claim wherein the shaft is provided with a rounded, blunted end which, in use, is inserted into the urethra.

7. A dosage form as claimed in any preceding claim characterised by a terminal portion (3; 13; 23; 32) at one end of the shaft adapted to prevent complete insertion of the dosage form in the urethra.

8. A dosage form as claimed in claim 7 wherein the terminal portion (3; 13; 23; 32) comprises a tubular cap (23) which is dimensioned to enclose the outer surface of the glans.

9. A dosage form as claimed in claim 8 wherein the tubular cap (23) also encloses a portion of the shaft of the penis juxtaposed the glans.

10. A dosage form as claimed in claim 8 or claim 9 wherein the composition (24) containing the therapeutic agent is also coated on a penis-contacting surface of the tubular cap; the arrangement being such that, in use, the therapeutic agent on the cap (23) is delivered transdermally to the penis.

11. A dosage form as claimed in any preceding claim wherein the shaft defines a bore which accommodates a piston (29), the composition being retained in the bore, such that when the shaft is inserted into the urethra the piston can slide in the bore and dislodge the composition into the urethra.

12. A dosage form as claimed in claim 11 further comprising a preventing means between the shaft and the piston (29) for preventing accidental operation of the piston.

13. A dosage form as claimed in claim 12 wherein said preventing means comprises a frangible bond (35) which resists relative motion of the shaft and the piston until a predetermined force is applied to break the frangible bond.

14. A dosage form as claimed in any of claims 11 to 13 wherein the piston (29) is provided with a plunger (30) juxtaposed the other end of the shaft, which other end of the shaft is provided with a cap portion (33) defining a receptacle (34) which can accommodate said plunger (30); the piston (29) being rotatable in the bore between a position in which the plunger butts on the cap portion so as to prevent operation of the piston (28) to deliver the unit dosage and another position in which the plunger (30) is aligned with the receptacle (34) so as to permit operation of the piston.

15. A dosage form as claimed in any preceding claim wherein said composition comprises a dispersant selected from PEG, propylene glycol, glycerine, polyvinyl pyrrolidone (PVP), polyvinyl alcohol (PVA) or hydroxy alkyl celluloses.

16. A dosage form as claimed in any preceding claim wherein said composition comprises PEG as a dispersant, said PEG having a molecular weight(s) such that the composition is solid at ambient temperature and melts when delivered in the urethra.

17. A dosage form as claimed in any preceding claim wherein the composition also contains a urethral permeation enhancer for the therapeutic agent.

18. A dosage form as claimed in claim 17 wherein said urethra permeation enhancer is selected from glycol monolaurate and polyethylene glycol monolaurate.

19. A dosage form as claimed in any preceding claim wherein the therapeutic agent is a vasoconstrictor, a vasodilator or an anti-inflammatory agent.

20. A dosage form as claimed in any of claims 1 - 18 wherein the therapeutic agent is selected from nitrates, long and short acting α-blockers, calcium blockers, ergot alkaloids, chlorpromazine, haloperidol, yohimbine, natural and synthetic vasoactive prostaglandins and their analogs, vasoactive intestinal peptides, dopamine agonists, opioid antagonists and mixtures thereof.

21. A dosage form as claimed in any of claims 1 - 18 wherein the therapeutic agent is selected from PGE₁, papaverine, phentolamine, prazosin, doxazosin and mixtures thereof.

22. A dosage form as claimed in any of claims 1 - 18 wherein the therapeutic agent is selected from α-agonists, β-blockers and mixtures thereof.

23. A dosage form as claimed in any of claims 1 - 18 wherein the agent is therapeutically active with respect to the treatment of erectile dysfunction.

24. A dosage form as claimed in any of claims 1 - 18 wherein the agent is selected from agents for the treatment of impotence, priapism and/or Peyronie's syndrome.

25. A dosage form as claimed in any of claims 1 - 18 wherein the therapeutic agent is one to which the urethra is permeable and which enters the blood supplying the corpus cavernosum.

26. A dosage form as claimed in any of claims 1 - 18 wherein the therapeutic agent is selected from 10 - 1000 µg of PGE₁, about 1 - 20 mg of papaverine, about 50 - 1000 µg phentolamine, about 50 - 1000 µg of prazosin, about 50 - 1000 µg of doxazosin and mixtures thereof.

27. A dosage form as claimed in any of claims 1 - 18 wherein the therapeutic agent is PGE₁.

28. A dosage form as claimed in any of claims 1 - 18 wherein the therapeutic agent is a mixture of PGE₁ and an α-blocker.

29. A dosage form as claimed in any of claims 1 - 18 wherein the therapeutic agent is a mixture of PGE₁ and prazosin.

30. An assembly comprising a dosage form as claimed in any preceding claim and a receptacle (15) therefor, wherein the receptacle can accommodate the shaft (2; 12; 22; 28) and the dosage form is provided with a plug portion (13) which can form a push-fit in an end of the receptacle.

31. An assembly as claimed in claim 31 wherein the plug portion (13) is provided with sliding seal means (13a) which can form a sliding seal with the end of the receptacle (15).

## Patentansprüche

1. Dosiereinheit (1; 10; 20; 25) zur Behandlung von Erektionsversagen, **gekennzeichnet** durch
a) einen Schaft (2; 12; 22; 28) mit einer Länge von 2 bis 12 cm, der in die Harnröhre eines männlichen Patienten eingeführt werden kann;
b) eine Zusammensetzung umfassend eine vorgewählte therapeutische Dosis eines therapeutischen Wirkstoffes zur Behandlung von Erektionsversagen, wobei die Zusammensetzung in einem Hohlraum in dem Ende des Schaftes angeordnet ist, das in die Harnröhre eingeführt wird,
c) wobei der Schaft und die Zusammensetzung so geformt bzw. angeordnet sind, daß beim Einführen des Schaftes in die Harnröhre die Zusammensetzung im wesentlichen hinter dem Punkt abgegeben wird, an dem der Übergang von dem epidermalen Charakter der Eichel abgeschlossen ist; und
d) Abgabemittel (29), die wahlweise betätigbar sind, um die Zusammensetzung aus dem Hohlraum in die Harnröhre abzugeben.

2. Dosiereinheit (1; 10; 20; 25) zur Behandlung von Erektionsversagen, **gekennzeichnet** durch
a) einen Schaft (2; 12; 22; 28) mit einer Länge von 2 bis 12 cm, der in die Harnröhre eines männlichen Patienten eingeführt werden kann;
b) eine Schicht auf mindestens einem Abschnitt des Schaftes, wobei die Schicht eine Zusammensetzung mit einer vorgewählten therapeutischen Dosis eines therapeutischen Wirkstoffes zur Behandlung von Erektionsversagen umfaßt; und
c) wobei der Schaft und die Schicht so ausgebildet und angeordnet sind, daß beim Einführen des Schaftes in die Harnröhre die Zusammensetzung im wesentlichen hinter dem Punkt abgegeben wird, an dem der Übergang von dem epidermalen Charakter der Eichel abgeschlossen ist.

3. Dosiereinheit nach Anspruch 1, wobei die Zusammensetzung ferner ein Dispersionsmittel für den therapeutischen Wirkstoff umfaßt.

4. Dosiereinheit nach Anspruch 1, 2 oder 3, wobei die Gesamtmenge der Zusammensetzung 100 mg nicht überschreitet.

5. Dosiereinheit nach einem der vorangegangenen Ansprüche, wobei die Gesamtmenge der Zusammensetzung 50 mg nicht überschreitet.

6. Dosiereinheit nach einem der vorangegangenen Ansprüche, wobei der Schaft mit einem abgerundeten stumpfen Ende versehen ist, das bei Gebrauch in die Harnröhre eingeführt wird.

7. Dosiereinheit nach einem der vorangegangenen Ansprüche, **gekennzeichnet** durch einen Endabschnitt (3; 13; 23; 32) an einem Ende des Schaftes, das dazu geeignet ist, das vollständige Einführen der Dosiereinheit in die Harnröhre zu verhindern.

8. Dosiereinheit nach Anspruch 7, wobei der Endabschnitt (3; 13; 23; 32) eine rohrförmige Kappe (23) umfaßt, die so bemessen ist, daß sie die Außenfläche der Eichel einschließt.

9. Dosiereinheit nach Anspruch 8, wobei die rohrförmige Kappe (23) auch einen der Eichel benachbarten Abschnitt des Penisschaftes einschließt.

10. Dosiereinheit nach Anspruch 8 oder 9, wobei die Zusammensetzung (24), welche den therapeutischen Wirkstoff enthält, auch auf eine mit dem Penis in Kontakt tretende Fläche der rohrförmigen Kappe aufgetragen ist, wobei die Anordnung so getroffen ist, daß im Gebrauch der therapeutische Wirkstoff auf der Kappe (23) an den Penis transdermal abgegeben wird.

11. Dosiereinheit nach einem der vorangegangenen Ansprüche, wobei der Schaft eine Bohrung begrenzt, die einen Kolben (29) aufnimmt, wobei die Zusammensetzung in der Bohrung enthalten ist, so daß nach dem Einführen des Schaftes in die Harnröhre der Kolben in der Bohrung gleiten kann und die Zusammensetzung in die Harnröhre schiebt.

12. Dosiereinheit nach Anspruch 11, wobei eine Sperreinrichtung zwischen dem Schaft und dem Kolben (29) vorgesehen ist, um eine zufällige Betätigung des Kolbens zu verhindern.

13. Dosiereinheit nach Anspruch 12, wobei die Sperreinrichtung eine brechbare Verbindung (35) umfaßt, die einer Relativbewegung des Schaftes und des Kolbens widersteht, bis eine vorgegebene Kraft aufgewandt wird, um die Verbindung aufzubrechen.

14. Dosiereinheit nach einem der Ansprüche 11 bis 13, wobei der Kolben (29) mit einem Kolbenkopf (30) versehen ist, der dem anderen Ende des Schaftes benachbart ist, wobei dieses andere Ende des Schaftes mit einem Kappenabschnitt (33) versehen ist, der einen Aufnahmehohlraum (34) zur Aufnahme des Kolbenkopfes (30) begrenzt, und wobei der Kolben (29) in der Bohrung zwischen einer Stellung, in welcher der Kolbenkopf an dem Kappenabschnitt anstößt, um die Betätigung des Kolbens (28) zur Abgabe der Dosiseinheit zu verhindern, und einer weiteren Stellung drehbar ist, in welcher der Kolbenkopf (30) mit dem Aufnahmehohlraum (34) fluchtet, um so eine Betätigung des Kolbens zu ermöglichen.

15. Dosiereinheit nach einem der vorangegangenen Ansprüche, wobei die Zusammensetzung ein Dispersionsmittel enthält, das aus PEG, Propylenglykol, Glycerin, Polyvinylpyrrolidon (PVP), Polyvinylalkohol (PVA) oder Hydroxyalkylcellulosen ausgewählt wurde.

16. Dosiereinheit nach einem der vorangegangenen Ansprüche, wobei die Zusammensetzung PEG als Dispersionsmittel enthält, wobei dieses PEG ein Molekulargewicht hat, das so gewählt ist, daß die Zusammensetzung bei Raumtemperatur fest ist und bei Abgabe an die Harnröhre schmilzt.

17. Dosiereinheit nach einem der vorangegangenen Ansprüche, wobei die Zusammensetzung auch ein Mittel enthält, das die Durchlässigkeit der Harnröhre für den therapeutischen Wirkstoff verstärkt.

18. Dosiereinheit nach Anspruch 17, wobei das die Durchlässigkeit der Harnröhre verstärkende Mittel aus Glykolmonolaurat oder Polyethylenglykolmonolaurat ausgewählt wird.

19. Dosiereinheit nach einem der vorangegangenen Ansprüche, wobei der therapeutische Wirkstoff ein Vasokonstriktor, ein Vasodilator oder ein entzündungshemmender Wirkstoff ist.

20. Dosiereinheit nach einem Ansprache 1 bis 18, wobei der therapeutische Wirkstoff ausgewählt wird aus Nitraten, lang oder kurz wirkenden α-Blockern, Calciumblockern, Mutterkornalkaloiden, Chlorpromazin, Haloperidol, Yohimbin, natürlichen oder synthetischen vasoaktiven Prostaglandinen und ihren Analogen, vasoaktiven Verdauungspeptiden, Dopaminagonisten, opioiden Antagonisten und Mischungen hiervon.

21. Dosiereinheit nach einem der Ansprüche 1 bis 18, wobei der therapeutische Wirkstoff ausgewählt wird aus PGE₁, Papaverin, Phentolamin, Prazosin, Doxazosin und Mischungen hiervon.

22. Dosiereinheit nach einem der Ansprüche 1 bis 18, wobei der therapeutische Wirkstoff ausgewählt wird aus α-Agonisten, β-Blockern und Mischungen hiervon.

23. Dosiereinheit nach einem der Ansprüche 1 bis 18, wobei der Wirkstoff bei der Behandlung von Erektionsversagen therapeutisch aktiv ist.

24. Dosiereinheit nach einem der Ansprüche 1 bis 18, wobei der Wirkstoff ausgewählt wird aus Wirkstoffen zur Behandlung von Impotenz, Priapismus und/oder Peyronie's Syndrom.

25. Dosiereinheit nach einem der Ansprüche 1 bis 18, wobei der therapeutische Wirkstoff ein solcher ist, für den die Harnröhre durchlässig ist und der in das den Schwellkörper versorgende Blut gelangt.

26. Dosiereinheit nach einem der Ansprüche 1 bis 18, wobei der therapeutische Wirkstoff ausgewählt wird aus 10 bis 1000 µg von PGE₁, ca. 1 bis 20 mg Papaverin, ca. 50 bis 1000 µg Phentolamin, ca. 50 bis 1000 µg Prazosin, ca. 50 bis 1000 µg Doxazosin und Mischungen hiervon.

27. Dosiereinheit nach einem der Ansprüche 1 bis 18, wobei der therapeutische Wirkstoff PGE₁ ist.

28. Dosiereinheit nach einem der Ansprüche 1 bis 18, wobei der therapeutische Wirkstoff eine Mischung PGE₁ und einem α-Blocker ist.

29. Dosiereinheit nach einem der Ansprüche 1 bis 18, wobei der therapeutische Wirkstoff eine Mischung von PGE₁ und Prazosin ist.

30. Anordnung umfassend eine Dosiereinheit nach einem der vorangegangenen Ansprüche und einen Aufnahmebehälter (15) hierfür, wobei der Behälter den Schaft (2; 12; 22; 28) aufnehmen kann und die Dosiereinheit mit einem Steckerabschnitt (13) versehen ist, der in ein Ende des Behälters eingesteckt werden kann.

31. Anordnung nach Anspruch 30, wobei der Steckerabschnitt (13) mit einem Gleitdichtmittel (13a) versehen ist, das eine Gleitdichtung mit dem Ende des Behälters (15) bilden kann.

## Revendications

1. Dispositif pour l'administration d'un médicament dit "forme de dosage" (1 ; 10 ; 20 ; 25) pour le traitement des troubles de l'érection caractérisé par :
a) une tige (2 ; 12 ; 22 ; 28) de 20 à 120 mm de longueur pouvant être insérée dans l'urètre d'un patient de sexe masculin ; et
b) une composition contenant une dose thérapeutique prédéfinie d'un agent thérapeutique pour le traitement des troubles de l'érection, placée dans une cavité se trouvant à l'extrémité de la tige insérée dans l'urètre ;
c) les dites tige et composition sont conçues et agencées de telle façon que lors de l'insertion de la tige dans l'urètre, la composition est libérée en grande partie au-delà du point auquel le gland n'a plus de caractère épidermique.
d) des moyens (29) de libération pouvant être sélectivement actionnés pour libérer la composition de la cavité dans l'urètre.

2. Dispositif pour l'administration d'un médicament (1 ; 10 ; 20 ; 25) pour le traitement des troubles de l'érection caractérisé par :
a) une tige (2 ; 12 ; 22 ; 28) de 20 à 120 mm de longueur pouvant être insérée dans l'urètre d'un patient de sexe masculin ; et
b) un enrobage recouvrant, en partie au moins, la tige, ledit enrobage contenant une composition comportant une dose thérapeutique prédéfinie d'un agent thérapeutique pour le traitement des troubles de l'érection ; et
c) lesdits tige et enrobage sont conçus et agencés de telle façon que lors de l'insertion de la tige dans l'urètre, la composition est libérée en grande partie au-delà du point auquel le gland n'a plus de caractère épidermique.

3. Dispositif pour l'administration d'un médicament selon la revendication 1, dans lequel ladite composition comprend également un agent dispersant pour l'agent thérapeutique.

4. Dispositif pour l'administration d'un médicament selon la revendication 1, la revendication 2 ou la revendication 3 et une quantité totale de composition ne dépassant pas 100 mg.

5. Dispositif pour l'administration d'un médicament selon l'une quelconque des revendications précédentes, dans lequel la quantité totale de composition ne dépasse pas 50 mg.

6. Dispositif pour l'administration d'un médicament selon l'une quelconque des revendications précédentes, dans lequel la tige est munie d'une extrémité arrondie et émoussée qui est, en cours d'utilisation, insérée dans l'urètre.

7. Dispositif pour l'administration d'un médicament selon l'une quelconque des revendications précédentes, caractérisé par une partie terminale (3 ; 13 ; 23 ; 32) située à une extrémité de la tige, adaptée de façon à empêcher l'insertion complète du dispositif pour l'administration d'un médicament dans l'urètre.

8. Dispositif pour l'administration d'un médicament selon la revendication 7, dans lequel la partie terminale (3 ; 13 ; 23 ; 32) comprend un capuchon tubulaire (23) dimensionné de façon à recouvrir la surface externe du gland.

9. Dispositif pour l'administration d'un médicament selon la revendication 8, dans lequel le capuchon tubulaire (23) recouvre également une partie de la tige du pénis juxtaposée au gland.

10. Dispositif pour l'administration d'un médicament selon la revendication 8 ou la revendication 9, dans lequel la composition (24) contenant l'agent thérapeutique est également déposée sur une surface du capuchon tubulaire en contact avec le pénis ; l'agencement étant tel que, en cours d'utilisation, l'agent thérapeutique situé sur le capuchon (23) est administré par voie transdermique dans le pénis.

11. Dispositif pour l'administration d'un médicament selon l'une quelconque des revendications précédentes, dans lequel la tige definit un axe creux permettant le passage d'un piston (29), la composition étant retenu dans l'axe creux, de telle sorte que, lorsque la tige est insérée dans l'urètre, le piston peut coulisser dans l'axe creux et pousser la composition dans l'urètre.

12. Dispositif pour l'administration d'un médicament selon la revendication 11, comprenant en outre des moyens préventifs situés entre la tige et le piston, visant à éviter toute poussée accidentelle du piston (29).

13. Dispositif pour l'administration d'un médicament selon la revendication 12, dans lequel lesdits moyens préventifs comprennent une attache cassable (35) qui résiste au mouvement relatif de la tige et du piston jusqu'à ce qu'une force prédéfinie soit appliquée pour rompre l'attache cassable.

14. Dispositif pour l'administration d'un médicament selon les revendications 11 à 13, dans lequel le piston (29) est muni d'un poussoir (30) juxtaposé à l'autre extrémité de la tige, ladite autre extrémité de la tige étant munie d'une partie de capuchon (33) définissant un réceptacle (34) pouvant accueillir ledit poussoir (30) ; le piston (29) pouvant tourner dans l'axe creux depuis une position dans laquelle le poussoir bute contre la partie de capuchon de façon à empêcher la poussée du piston (28) libérant la dose unitaire et une autre position dans laquelle le poussoir (30) est aligné avec le réceptacle (34) de façon à permettre le mouvement du piston.

15. Dispositif pour l'administration d'un médicament selon l'une quelconque des revendications précédentes, dans lequel ladite composition comprend un agent dispersant choisi parmi le PEG, le propylène glycol, la glycérine, la polyvinylpyrrolidone (PVP), l'alcool polyvinylique (PVA) ou des hydroxyalkylcelluloses.

16. Dispositif pour l'administration d'un médicament selon l'une quelconque des revendications précédentes, dans lequel ladite composition comprend du PEG comme agent dispersant, ledit PEG ayant une masse moléculaire telle que la composition est solide à la température ambiante et fond lorsqu'elle est libérée dans l'urètre.

17. Dispositif pour l'administration d'un médicament selon l'une quelconque des revendications précédentes, dans lequel la composition contient également un agent qui améliore la perméation urétrale de l'agent thérapeutique.

18. Dispositif pour l'administration d'un médicament selon la revendication 17, dans lequel ledit agent qui améliore la perméation urétrale est choisi parmi le monolaurate de glycol ou le monolaurate de polyéthylène glycol.

19. Dispositif pour l'administration d'un médicament selon l'une quelconque des revendications précédentes, dans lequel l'agent thérapeutique est un agent vasoconstricteur, vasodilatateur ou anti-inflammatoire.

20. Dispositif pour l'administration d'un médicament selon l'une quelconque des revendications 1 à 18, dans lequel l'agent thérapeutique est choisi parmi les nitrates, les α-bloquants à action courte ou prolongée, les inhibiteurs calciques, les alcaloïdes de l'ergot, la chlorpromazine, l'halopéridol, la yohimbine, les prostaglandines vasoactives naturelles ou synthétiques et leurs analogues, les peptides intestinaux vasoactifs, les agonistes de la dopamine, les antagonistes opioïdes ou un mélange de ces produits.

21. Dispositif pour l'administration d'un médicament selon l'une quelconque des revendications 1 à 18, dans lequel l'agent thérapeutique est choisi parmi la PGE₁, la papavérine, la phentolamine, la prazosine, la doxazosine ou un mélange de ces produits.

22. Dispositif pour l'administration d'un médicament selon l'une quelconque des revendications 1 à 18, dans lequel l'agent thérapeutique est choisi parmi les α-agonistes, les β-bloquants ou un mélange de ces produits.

23. Dispositif pour l'administration d'un médicament selon l'une quelconque des revendications 1 à 18, dans lequel l'agent utilisé est actif au plan thérapeutique pour le traitement des troubles de l'érection.

24. Dispositif pour l'administration d'un médicament selon l'une quelconque des revendications 1 à 18, dans lequel l'agent thérapeutique est choisi parmi les agents pour le traitement de l'impuissance, du priapisme et/ou du syndrome de Peyronie.

25. Dispositif pour l'administration d'un médicament selon l'une quelconque des revendications 1 à 18, dans lequel l'agent thérapeutique est un agent qui traverse l'urètre et passe dans le sang circulant dans le corps caverneux.

26. Dispositif pour l'administration d'un médicament selon l'une quelconque des revendications 1 à 18, dans lequel l'agent thérapeutique est choisi parmi 10-1000 µg de PGE₁, environ 1-20 mg de papavérine, environ 50-1000 µg de phentolamine, environ 50-1000 µg de prazosine, environ 50-1000 µg de doxazosine et des mélanges de ces produits.

27. Dispositif pour l'administration d'un médicament selon l'une quelconque des revendications 1 à 18, dans lequel l'agent thérapeutique est du PGE₁.

28. Dispositif pour l'administration d'un médicament selon l'une quelconque des revendications 1 à 18, dans lequel l'agent thérapeutique est un mélange de PGE₁ et d'un α-bloquant.

29. Dispositif pour l'administration d'un médicament selon l'une quelconque des revendications 1 à 18, dans lequel l'agent thérapeutique est un mélange de PGE₁ et de prazosine.

30. Ensemble comprenant un dispositif pour l'administration d'un médicament selon l'une quelconque des revendications précédentes et un réceptacle (15) pour lui, dans lesquels le réceptacle peut accueillir la tige (2 ; 12 ; 22 ; 28) et le dispositif pour l'administration d'un médicament est muni d'une partie de bouchon (13) qui peut former un ajustage gras dans une extrémité du réceptacle.

31. Ensemble selon la revendication 31, dans lequel la partie de bouchon (13) est munie de moyens d'étanchéité coulissants (13a), qui peuvent former un joint coulissant avec l'extrémité du réceptacle (15).
